# EUROPEAN PATENT APPLICATION

(11) **EP 2 244 464 A2**
(43) Date of publication of application: **27.10.2010**
(21) Application number: 09702430.1
(22) Date of filing: 15.01.2009
(51) Int. Cl.: H04N 5/262

(54) **APPARATUS FOR ACQUIRING MULTILAYER PANORAMIC IMAGES AND METHOD FOR ACQUIRING PANORAMIC IMAGES**

(30) Priority: 15.01.2008 KR 20080004369
(71) Applicant: Vatech Co., Ltd, Gyeonggi-Do 445-810 (KR)
(72) Inventor: RO, Chang Joon, Seongnam Si Gyeonggi-Do 463-010 (KR); CHO, Hyo Sung, Wonju Si Gangwon-Do 220-040 (KR); CHOI, Sung Il, Wonju Si Gangwon-Do 220-090 (KR)
(74) Representative: Beck & Rössig European Patent Attorneys
(86) International application number: PCT/KR2009/000232
(87) International publication number: WO 2009/091203

(57) **Abstract**

The present invention relates to a panoramic image acquisition apparatus and method, and, more particularly, to a multilayer panoramic image acquisition apparatus and method, which is capable of acquiring images of a plurality of image layers at a single time by synchronizing sections where X-rays are radiated onto the plurality of image layers located in the areas of interest of a subject.

## Description

### Technical Field

The present invention relates to a panoramic image acquisition apparatus and method, and, more particularly, to a multilayer panoramic image acquisition apparatus and method, which is capable of acquiring images of a plurality of image layers at a single time by synchronizing sections where X-rays are radiated onto the plurality of image layers located in the areas of interest of a subject.

### Background Art

In general, a panoramic image is an image that enables a piece of scenery, which spreads out laterally, to be viewed at a glance, and is generally constructed by connecting several partially captured photos.

However, with regard to a panoramic image in the medical field, a panorama is generally constructed by reconstructing a three-dimensional image, which is obtained by sequentially scanning a specific section of a subject, into a two-dimensional (2D) plane image.

Furthermore, a Time Delay Integration (TDI) scan-type sensor is used as an image sensor used in a conventional panoramic image acquisition apparatus.

This is so, since a single image is constructed by receiving successively radiated X-rays and overlaying images on each other in each pixel of an image sensor, and therefore, it is impossible to establish two or more image layers.

Meanwhile, an image layer refers to a section on which the panoramic image acquisition apparatus is focused, and refers to a region which can be clearly viewed when an image is captured.

Accordingly, the conventional panoramic image acquisition apparatus cannot acquire a clear image when the area of interest of a subject to be photographed is not located in a preset image layer.

Furthermore, since the upper and lower jaws of a human person are not located in the same vertical surface, in order to clearly photograph the upper and lower jaws, there is the inconvenience of first locating and photographing the upper jaw in a preset image layer and then locating and photographing the lower jaw in the image layer by moving the human person closer to or further away from the X-ray light source.

### Disclosure

### Technical Problem

Accordingly, the present invention has been made while keeping in mind the above problems occurring in the prior art, and an object of the present invention is to provide a multilayer panoramic image acquisition apparatus and a panoramic image acquisition method which are capable of simultaneously acquiring images of a plurality of image layers, using one panoramic photographing operation.

### Technical Solution

In order to accomplish the above object, the present invention provides a multilayer panoramic image acquisition apparatus, including an X-ray light source for intermittently radiating X-rays onto a plurality of image layers where areas of interest of a subject are located, while moving around the subject along a specific trajectory, the X-ray light source radiating X-ray groups, i.e., groups of X-rays used to photograph the respective image layers; an image sensor configured to face the X-ray light source, to move to correspond to the X-ray light source and to acquire images of the image layers by sequentially receiving the X-rays having passed through the image layers, the image sensor acquiring frame images, i.e., unit images based on the respective X-rays; a synchronization module for radiating the X-ray groups, so that the X-ray groups can cover all sections of the image layers, preventing X-rays of each of the X-ray groups having passed through each of the image layers from overlapping each other, and performing synchronization, so that light reception operations of the image sensor can be performed in sections where the X-rays are radiated; and an image processing device for receiving the acquired frame images from the image sensor, extracting only images based on each of the image layers, and reconstructing the extracted images into a panoramic image of the image layer.

In a preferred embodiment, the X-ray light source radiates X-rays while rotating through a preset angle around a center of rotation between the X-ray light source and the image layers, the X-ray light source radiating the X-rays onto all sections of the image layers during an entire rotation.

In a preferred embodiment, the image layers include two image layers, any one of the image layers being located in an upper jaw of a human person, and a remaining image layer being located in a lower jaw.

In order to accomplish the above object, the present invention provides a multilayer panoramic image acquisition method, including a first step of providing an X-ray light source for radiating X-rays onto a plurality of image layers where areas of interest of a subject are located and an image sensor for receiving the X-rays having passed through the image layers, with the subject being placed between the X-ray light source and the image sensor; a second step of acquiring first sectional images of the respective image layers by radiating X-rays onto first sections, i.e., first side portions of the respective image layers; a third step of rotating the X-ray light source through a preset angle around a center of rotation between the X-ray light source and the image layers, and acquiring images of second sections of the image layers neighboring the first sections by radiating X-rays onto the second sections; a fourth step of acquiring images ranging up to images of n-th sections, i.e., remaining side portions of the image layers by sequentially photographing sections neighboring the second sections; and a fifth step of extracting sectional images of each of the image layers and reconstructing the sectional image into a panoramic image of each of the image layers; wherein the X-rays of the second to fourth steps are divided into X-ray groups, i.e., groups of X-rays used to photograph the respective image layers, X-rays of each of the X-ray groups are radiated not to overlap each other, while passing through each of the image layers, and all X-rays of the X-ray groups are radiated during the rotation of the X-ray light source.

In a preferred embodiment, the image layers are spaced apart from the X-ray light source by different distances, and include two image layers, any one of the image layers being located in an upper jaw of a human person, and a remaining image layer being located in a lower jaw.

### Advantageous Effects

As described above, according to the present invention, there is the effect of acquiring images of a plurality of image layers using one photographing operation by synchronizing the times at which X-rays are radiated.

Furthermore, according to the present invention, there is the effect of reducing the amount of X-rays to which a subject is exposed, by not using the scan method of continuously radiating X-rays but by using a method of intermittently radiating X-rays and then capturing frame images.

### Description of Drawings

Fig. 1 is a diagram showing a multilayer panoramic image acquisition apparatus according to an embodiment of the present invention;
Fig. 2 is a diagram showing a multilayer panoramic image acquisition method according to an embodiment of the present invention; and,
Fig. 3 is a diagram showing an image of a subject captured according to an embodiment of the present invention.

In the drawings according to the present invention, the same reference numerals are used to designate elements having substantially the same structures and functions.

### <Description of reference characters of principal elements in the drawings>

| | |
|---|---|
| 100: X-ray light source | 200: image sensor |
| 300: synchronization module | 400: image processing device |
| 500: display device | 10a: first image layer |
| 10b: second image layer | 100a: first X-ray group |
| 100b: second X-ray group | 10a', 10b': panoramic image |

### Best Mode

Embodiments of the present invention will be described in detail below with reference to the accompanying drawings.

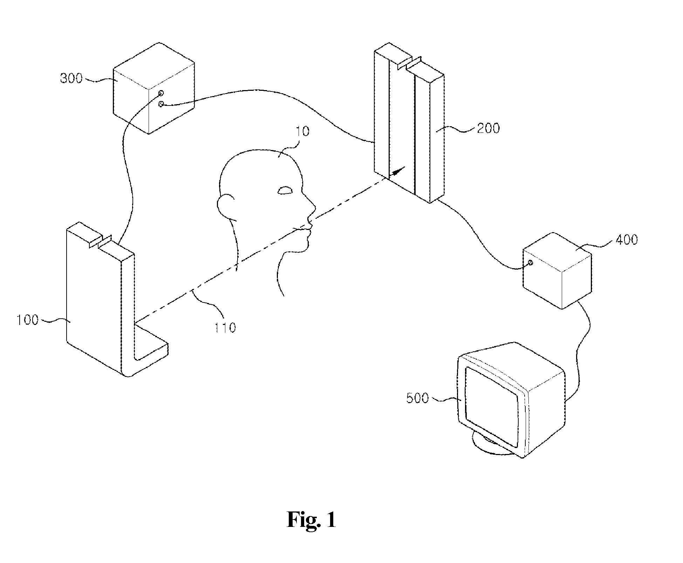

Fig. 1 is a diagram showing a multilayer panoramic image acquisition apparatus according to an embodiment of the present invention.

Referring to Fig. 1, a multilayer panoramic image acquisition apparatus according to an embodiment of the present invention includes an X-ray light source 100, an image sensor 200, a synchronization module 300, an image processing device 400, and a display device 500.

The X-ray light source 100 includes an X-ray tube (not shown) for generating X-rays, and radiates X-rays 110 onto a subject 10 using a slit (not shown).

Furthermore, the X-ray light source 100 intermittently radiates X-rays while moving around the subject 10 along a preset trajectory.

Furthermore, the X-ray light source 100 radiates X-rays 110 into image layers where the areas of interest of the subject 10 are located.

Here, the areas of interest refers to the inner portions of the subject 10 to be photographed, and refers to the upper and lower jaws of a human person in an embodiment of the present invention.

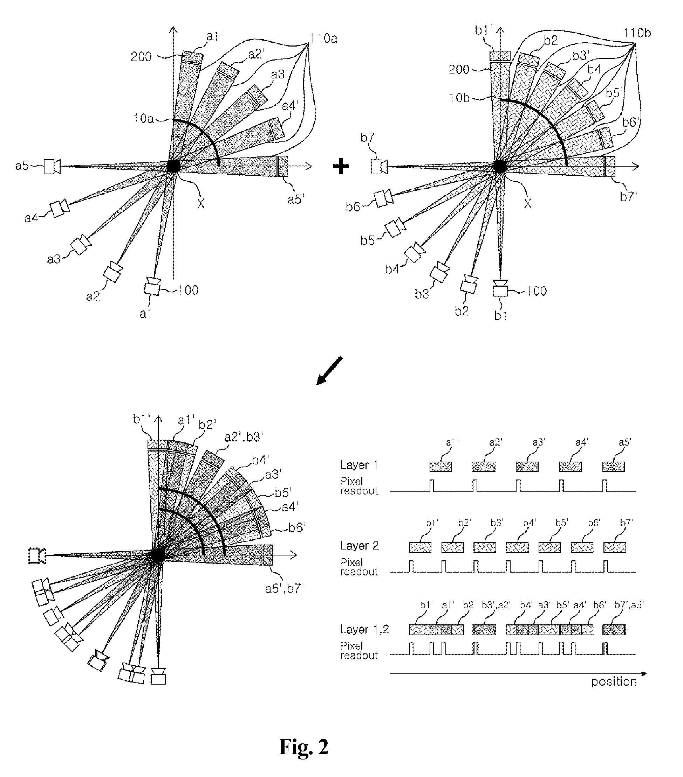

Meanwhile, a detailed description of the movement of the X-ray light source 100 will be given with reference to Fig. 2.

Furthermore, the image layers are a plurality of image layers, and are set to two image layers 10a and 10b in an embodiment of the present invention. The image layers 10a and 10b are located in the upper and lower jaws 11 and 12.

Furthermore, the X-rays 110 intermittently radiated by the X-ray light source 100 are divided into a first X-ray group 110a and a second X-ray group 110b, which are X-ray groups that are used to photograph the image layers 10a and 10b respectively.

So, the X-rays of the first X-ray group 110a are radiated to photograph the first image layer 10a, while the X-rays of the second X-ray group 110b are radiated to photograph the second image layer 10b.

The image sensor 200 is an area sensor having a specific area, and acquires images of the area of interest of subject 10, located in the image layers 10a and 10b, by receiving the X-rays 110 while facing the X-ray light source 100 and moving around the subject 10.

Furthermore, the image sensor 200 acquires a frame image, which is a unit image based on each of the intermittent X-rays 110.

So, the image sensor 200 is an image sensor of a size that covers part of the subject 10, and is a small area sensor of a size smaller than that of a conventional large area sensor (a Cephalo sensor) that covers the overall area of the subject 10 and photographs the overall area of the subject 10 at a single time.

In other words, the image sensor 200 acquires a frame-based image based on each intermittent X-ray, unlike the conventional Time Delay Integration (TDI) method in which a photographing is performed by continuous scanning.

Furthermore, the image sensor 200 may be formed by a CCD sensor or a CMOS sensor. However, the image sensor 200 may be any type of sensor as long as it can receive X-rays.

The synchronization module 300 synchronizes the time when the X-ray light source 100 radiates an X-ray 110 with the time when the image sensor 200 receives the X-ray 110.

Furthermore, the synchronization module 300 controls radiation to be performed in order to enable X-rays, included in the X-ray groups 110a and 110b radiated by the X-ray light source 100, to cover all the sections of the image layers 10a and 10b.

So, the synchronization module 300 functions to adjust the location or time of radiation, when the X-ray light source 100 is radiating the X-rays 110 onto the subject 10 while moving.

Furthermore, the synchronization module 300 controls the X-ray light source 100 to radiate X-rays, so that X-rays included in each of the X-ray groups 110a and 110b do not overlap each other, when the X-rays included in each of the X-ray groups 110a and 110b pass through each of the image layers 10a and 10b.

A detailed description will be given with reference to Fig. 2.

Furthermore, the synchronization module 300 is connected to the image sensor 200, and controls the light reception operation of the image sensor 200 to be performed in a section where the X-ray 110 has been radiated.

So, the image sensor 200 moves while facing the X-ray light source 200, and acquires images of the image layers 10a and 10b by performing light reception operations in accordance with the times where the intermittent X-rays are radiated.

The image processing device 400 receives acquired frame images from the image sensor 200, divides the images photographed based on the X-ray groups 110a and 110b according to the group, and reconstructs the images of the image layers 10a and 10b into a panoramic image.

The display device 500 displays panoramic images of the image layers 10a and 10b, reconstructed by the image processing device 400, to a user.

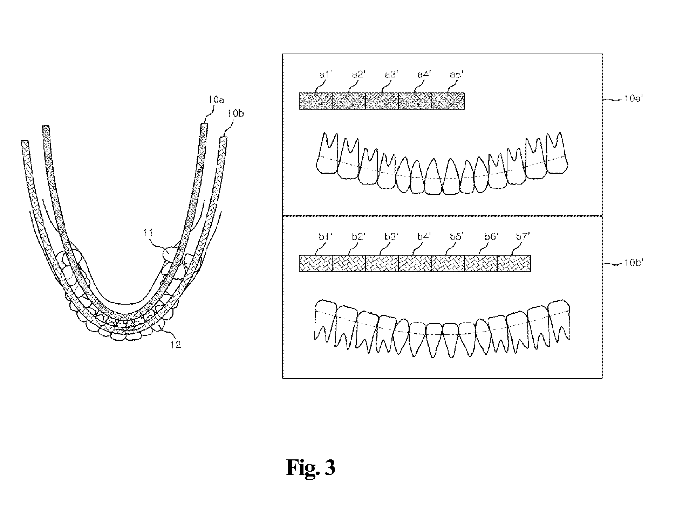

Fig. 2 is a diagram showing a multilayer panoramic image acquisition method, according to an embodiment of the present invention.̅ And, Fig. 3 is a diagram showing an image of a subject captured, according to an embodiment of the present invention.

Descriptions of elements, which are the same as those of Fig. 1, will be omitted below.

Referring to the drawings, the multilayer panoramic image acquisition method, according to an embodiment of the present invention, is performed by providing the X-ray light source 100 for radiating X-rays 110 onto a plurality of image layers 10a and 10b, where the areas of interest of the subject 10 are located and the image sensor 200 for receiving the X-rays 110 that have passed through the image layers 10a and 10b, with the subject 10 being placed between the X-ray light source and the image sensor.

Meanwhile, the areas of interest of the subject refer to regions, the images of which are desired to be photographed by a user: for example, the upper and lower jaws 10a and 10b in an embodiment of the present invention. The image layers 10a and 10b include two image layers 10a and 10b corresponding to the areas of interest. Of the image layers 10a and 10b, the first image layer 10a is located in the upper jaw 10a, and the second image layer 10b is located in the lowerjaw 10b.

The reason for this is that the upper and lower jaws 10a and 10b are not spaced apart by the same distance when viewed from the front.

In other words, since the upper jaw 10a of a typical human person is closer to the back area of the head than the lower jaws 10b is, the distances the X-rays of the X-ray light source 100 must pass through are different from each other, so that clear images cannot be acquired at the same time.

Thereafter, the X-ray light source 100 radiates X-rays into first sections, i.e., the first side portions of the image layers 10a and 10b, thereby, acquiring first sectional images b1' and a1', i.e., images of the first sections of the image layers 10a and 10b.

In a greater detail, the X-ray light source 100 radiates an X-ray at location b1, thereby, acquiring the first sectional image b1' of the second image layer 10b, rotates at a specific angle around the center of rotation x, and radiates an X-ray at location a1, thereby, acquiring the first sectional image a1' of the first image layer 10a.

However, the first sectional image a1' of the first image layer 10a and the first sectional image b1' of the second image layer 10b may be captured at the same time.

The reason for this is that, since from the point of view of the characteristics of a panoramic image, the capturing of all sectional images of the image layers 10a and 10b is enough, second sectional images b2' and a2' that are captured after the first sectional images b1' and a1', are captured in order to be connected to the first sectional images b1' and a1',̅ respectively.

Thereafter, the X-ray light source 100 moves to location b2, and acquires the second sectional image b2', i.e., an image of the second section neighboring the first section of the second image layer 10b.

Thereafter, the X-ray light source 100 moves to the location a2, and acquires the second sectional image a2', i.e., an image of the second section neighboring the first section of the first image layer 10a.

Thereafter, once images of sections neighboring the previously photographed sections of the image layers are successively acquired and then sectional images a5' and b7' of n-th sections, i.e., remaining side portions of the image layers 10a and 10b, are acquired, the photographing is terminated.

Meanwhile, the X-rays 110 are divided into a first X-ray group 110a and a second X-ray group 1 10b, i.e., X-ray groups used to photograph the image layers 10a and 10b, and the X-rays of each of the X-ray groups 1 10a and 110b pass through each of the image layers 10a and 10b while not overlapping each other. However, it will be apparent that the X-rays of different groups may overlap each other.

So, the synchronization module 300 synchronizes locations where the X-ray light source 100 radiates X-rays 110, and controls the light reception operation of the image sensor 200 to be performed in accordance with the location where the X-ray light source 100 radiates the X-rays 110.

Furthermore, in an embodiment of the present invention, sectional images b1', a1', b2', b3', a2', b4', a3', b5', a4', b6', b7', and a5' are sequentially acquired.

However, the sequence of acquisition and numbers of the sectional images may vary, depending on the distance between the image layers 10a and 10b.

Thereafter, the sectional images b1', a1', b2', b3', a2', b4', a3', b5', a4', b6', b7' and a5' are divided into and extracted as the sectional images a1', a2', a3', a4' and a5' of the first image layer 10a and the sectional images b1', b2', b3', b4', b5', b6' and b7', and are then reconstructed into the panoramic images 10a' and 10b' of the image layers 10a and 10b.

So, the panoramic image 10a' of the upper jaw 10a is obtained by connecting the sectional images a1 ∼ a5' of the first image layer 10a in a horizontal direction, and the panoramic image 10b' of the lower jaw 10b is obtained by connecting the sectional images b1 ∼ b7' of the second image layer 10b in a horizontal direction.

Accordingly, it is possible to acquire images of a plurality of image layers 10a and 10b, by radiating a minimum quantity of X-rays 110 onto the subj ect 10.

Although the configuration and operation of the present invention have been illustrated in conjunction with the above description and drawings, this is merely an example, and it will be apparent that various modifications and variations are possible within a range that does not depart from the scope of the present invention.

### Industrial Applicability

The present invention can acquire panoramic images of a plurality of image layers at a single time, and can be used for the acquisition of a variety of types of medical panoramic images as well as dental panoramic images.

## Claims

1. A multilayer panoramic image acquisition apparatus, comprising:
an X-ray light source for radiating X-rays onto a plurality of preset image layers where areas of interest of a subject are located while moving around the subject along a specific trajectory;
an image sensor configured to face the X-ray light source, to move to correspond to the X-ray light source, and to acquire images of the image layers;
a synchronization module for controlling the X-rays to be radiated onto all sections of each of the image layers, preventing X-rays having passed through each of the image layers from overlapping each other, and performing synchronization so that light reception operations of the image sensor can be performed while the X-rays are radiated; and
an image processing device for receiving the acquired images from the image sensor and reconstructing the acquired images into panoramic images of the respective image layers.

2. The multilayer panoramic image acquisition apparatus according to claim 1, wherein the X-ray light source radiates X-rays while rotating through a preset angle around a center of rotation between the X-ray light source and the image layers, and radiates the X-rays onto all sections of the image layers during an entire rotation.

3. The multilayer panoramic image acquisition apparatus according to claim 1 or 2, wherein the image layers include two image layers, any one of the image layers being located in an upper jaw of a human person, and a remaining image layer being located in a lower jaw.

4. A multilayer panoramic image acquisition method, comprising:
a first step of providing an X-ray light source for radiating X-rays onto a plurality of image layers where areas of interest of a subject are located and an image sensor for receiving the X-rays having passed through the image layers, with the subject being disposed between the X-ray light source and the image sensor;
a second step of acquiring first sectional images of the respective image layers by radiating X-rays onto first sections, that is, first side portions of the respective image layers;
a third step of acquiring images ranging from second sectional images of the image layers neighboring the first sectional images of the image layers to n-th sectional images, that is, images of remaining sides of the image layers while rotating the X-ray light source and the image sensor; and
a fourth step of reconstructing the sectional images of each of the image layers into a panoramic image of each of the image layers,
wherein the X-rays of the second and third steps are radiated not to overlap each other, while passing through each of the image layers, and are radiated onto all sections of each of the image layers during the rotation of the X-ray light source.

5. The multilayer panoramic image acquisition method according to claim 4, wherein the image layers are spaced apart from the X-ray light source by different distances, and include two image layers, any one of the image layers being located in an upper jaw of a human person, and a remaining image layer being located in a lower jaw.

6. A multilayer panoramic image acquisition apparatus, comprising:
an X-ray light source for intermittently radiating X-rays onto a plurality of image layers where areas of interest of a subject are located while moving around the subject along a specific trajectory, the X-ray light source radiating X-ray groups, that is, groups of X-rays used to photograph the respective image layers;
an image sensor configured to face the X-ray light source, to move to correspond to the X-ray light source, and to acquire images of the image layers by sequentially receiving the X-rays having passed through the image layers, the image sensor acquiring frame images, that is, unit images based on the respective X-rays;
a synchronization module for controlling the radiation of the X-ray groups, so that the X-ray groups can cover all sections of the image layers, preventing X-rays of each of the X-ray groups having passed through each of the image layers from overlapping each other, and performing synchronization so that light reception operations of the image sensor can be performed in sections where the X-rays are radiated; and
an image processing device for receiving the acquired frame images from the image sensor, extracting images of the same X-ray group, respectively, and reconstructing the extracted images into a panoramic image of the image layer.

7. The multilayer panoramic image acquisition apparatus according to claim 6, wherein the X-ray light source radiates X-rays while rotating through a preset angle around a center of rotation between the X-ray light source and the image layers, the X-ray light source radiating the X-rays onto all sections of the image layers during an entire rotation.

8. The multilayer panoramic image acquisition apparatus according to claim 6 or 7, wherein the image layers include two image layers, any one of the image layers being located in an upper jaw of a human person, and a remaining image layer being located in a lower jaw.

9. A multilayer panoramic image acquisition method, comprising:
a first step of providing an X-ray light source for radiating X-rays onto a plurality of image layers where areas of interest of a subject are located and an image sensor for receiving the X-rays having passed through the image layers, with the subject being placed between the X-ray light source and the image sensor;
a second step of acquiring first sectional images of the respective image layers by radiating X-rays onto first sections, that is, first side portions of the respective image layers;
a third step of rotating the X-ray light source through a preset angle around a center of rotation between the X-ray light source and the image layers, and acquiring images of second sections of the image layers neighboring the first sections by radiating X-rays onto the second sections;
a fourth step of acquiring images ranging up to images of n-th sections, that is, remaining side portions of the image layers by sequentially photographing sections neighboring the second sections; and
a fifth step of extracting sectional images of each of the image layers and reconstructing the sectional image into a panoramic image of each of the image layers;
wherein the X-rays of the second to fourth steps are divided into X-ray groups, that is, groups of X-rays used to photograph the respective image layers, X-rays of each of the X-ray groups are radiated not to overlap each other while passing through each of the image layers, and all X-rays of the X-ray groups are radiated during an entire rotation of the X-ray light source.

10. The multilayer panoramic image acquisition method according to claim 9, wherein the image layers are spaced apart from the X-ray light source by different distances, and include two image layers, any one of the image layers being located in an upper jaw of a human person, and a remaining image layer being located in a lower jaw.
